# EUROPEAN PATENT SPECIFICATION

(11) **EP 1 087 756 B1**
(45) Date of publication and mention of the grant of the patent: **05.08.2009**
(21) Application number: 99930433.0
(22) Date of filing: 18.06.1999
(51) Int. Cl.: A61K 9/19, A61K 35/36, C08L 89/06

(54) **PARTICULATE ACELLULAR TISSUE MATRIX**
TEILCHENFÖRMIGE ZELLFREIE GEWEBEMATRIX
MATRICE TISSULAIRES ACELLULAIRES PARTICULAIRES

(30) Priority: 19.06.1998 US 89865 P
(43) Date of publication of application: 04.04.2001
(73) Proprietor: LIFECELL CORPORATION, Branchburg, NJ 08876-3876 (US)
(72) Inventor: GRIFFEY, Edward, S., The Woodlands, TX 77381 (US); LIVESEY, Stephen, A., Eltham, VIC 3095 (AU); SCHIFF, Charles, M., The Woodlands, TX 77381 (US); BOERBOOM, Lawrence, E., The Woodlands, TX 77381 (US)
(74) Representative: Eccetto, Mauro
(86) International application number: PCT/US1999/013861
(87) International publication number: WO 1999/065470

(56) References cited:
- WO-A-94/03584
- US-A- 4 776 173
- US-A- 5 256 140
- US-A- 5 275 826
- US-A- 5 695 998
- US-A- 5 893 888

## Description

### BACKGROUND OF THE INVENTION

Human, animal and synthetic materials are currently used in medical procedures to augment tissue or repair or correct tissue defects. To be optimum, such materials should not migrate and should promote the regeneration of normal tissue, repopulating with the host's cells, revascularizing, and integrating with the patient's own tissue without triggering an inflammatory response that results in the degradation or resorption of the material. Additionally, the manner of delivery of such material, e.g. by surgical procedure or by injection, may significantly affect the clinical applications of the material, the ease of use by the physician and the cost of the procedure.

Injectable collagen and other materials have been used clinically for a wide variety of pathological and cosmetic applications in the fields of reconstructive surgery, dermatology, oncology, otolaryngology and urology. Currently, the most widely used form of injectable collagen is derived from crosslinked bovine Type I collagen. In human clinical applications the effect of this xenogenic transplant is resorption by the human host. Patients receiving these xenogenic grafts are susceptible to an immune response to the animal collagen, requiring prescreening for existing antibodies. Examples of such materials may be found in U.S. Patent Numbered: 4,582,640; 5,104,957; 5,728,752; and 5.739,176.

Human collagen that may be injected is currently available and sold under the tradenames Autologen^{®} and Dermologen^{®} and is manufactured by Collagenesis. This material it typically derived from autologous collagen obtained during elective surgery or allogenic collagen from cadavers. The starting material is dissociated by mechanical means and chemically treated to remove all noncollagenous proteins. The collagen is treated with additional chemicals to mask or crosslink the adverse effects of these damaged and exposed collagen fibers. More information with regard to this technology may be found in U.S. Patent Numbers: 4,969,912 and 5,332,802.

AlloDerm^{®}, produced by LifeCell Corporation, is an acellular tissue matrix which is produced from normal human skin using processing techniques established to remove the epidermis and cells within the dermis without significantly altering the normal biochemistry and molecular architecture of the connective tissue matrix. The resulting product is in a freeze-dried form allowing extended shelf life and ease of shipping without degradation or loss of the normal tissue matrix components. AlloDerm^{®} is used clinically to repair or replace damaged or inadequate tissues. Reported applications for AlloDerm^{®} include: full thickness bum injury, replacement of lost gingiva due to periodontal disease, reconstructive surgical applications involving the replacement of lost tissue or restoration of normal surface contours of skin damaged due to injury or aging neurosurgical application to replace lost dura and in urological applications such as bladder slings and pelvic floor reconstruction.. AlloDerm^{®} has been reported to integrate at the graft site where it is rapidly repopulated with the normal milieu of host cells. A reported benefit of AlloDerm^{®} is that it maintains the structure and biochemistry of the tissue matrix, promoting normal tissue regeneration. Studies have indicated that AlloDerm^{®} retains decorin, hyaluronic acid, chondroitin sulfates, nidogen, growth factors and other biochemical proteins present in normal soft tissues. Additionally, AlloDerm^{®} is reported to contain the basement membranes of vascular channels and the orientation of elastin and collagen fibers of the starting dermal tissue. For these reasons it is believed that the structure and biochemistry of the AlloDerm^{®} matrix promotes tissue regeneration. Reducing sheet AlloDerm^{®} to a particulate suitable for injection should extend the beneficial properties of AlloDerm^{®} to several new applications.

Methods presently used to produce currently available injectable collagen materials include mechanical disruption of the starting material in its wet, hydrated state. However, when such processes are carried out on intact autograft, allograft or xenograft tissue, damage to the matrix occurs such that following transplantation a foreign body response and rapid resorption of the tissue matrix occurs. Further, microscopic and histological analysis of material processed in such a manner exhibit mechanical disruption of the collagen fibers. Mechanical disruption of dried human or animal tissue at non-cryogenic temperatures is believed to create a similar disruption of the collagen fibers, resulting in a foreign body response by the recipient and resorption of the material.

Thus there exists an unmet need for a method of making an intact particulate acellular tissue matrix from acellular tissues.

PCT Application number WO 94/03584 discloses a method for producing matrix particulates which are used for tissue reconstruction. The manufacturing process includes a fragmentation step at cryogenic temperatures.

### SUMMARY OF THE INVENTION

According to the present invention, the use of a product consisting in a particulate tissue matrix according to claim 1 is provided.

The present invention is generally directed to a method of processing an acellular tissue matrix to give a particulate acellular tissue matrix. A general embodiment of the method of the present invention includes the steps of: cryofracturing the dry acellular tissue matrix strips at cryogenic temperatures; and separating the resulting particles by size at cryogenic temperatures. In a prefered embodiment, the method of the present invention includes: cutting sheets of dry acellular tissue matrix into strips; cryofracturing the dry acellular tissue matrix strips at cryogenic temperatures; separating the resulting particles by size at cryogenic temperatures; and freeze drying the fraction of particles desired size to remove any moisture that may have been absorbed to give a dry particulate acellular tissue matrix. Rehydration of the dry particulate acellular tissue matrix may take place just prior to use.

It is generally preferred that the cryofracturing be carried out at temperatures below 0° C and preferably the temperature should be below -50°C and more preferably should be below -100° C. Commercially available refrigerants can be used to achieve such temperatures, which may include halocarbon refrigerants, liquid carbon dioxide, liquid nitrogen, liquid argon, liquid helium and other similar such well known non-chemically reactive and thus inert and non-toxic refrigerants. The separation of the resulting particles should also be carried out at cryogenic temperatures utilizing a series of metal mesh screens suitably sized for the particle range desired. In one preferred embodiment the screens are selected so as to isolate particles having a size of 1 µm (micron) to 900 µm (microns) and preferably screens are selected to isolate particles having a size from 30 µm (microns) to 800 µm (microns). The present invention also encompasses the product of the above described processes.

These and other features of the present invention are more fully set forth in the following description of illustrative embodiments of the invention.

### BRIEF DESCRIPTION OF THE DRAWINGS

The description is presented with reference to the accompanying drawings in which:
FIG. 1 is an illustration of a bundle of collagen fibers which have been cryofractured in accordance with the present invention.
FIG. 2 is an illustration of a bundle of collagen fibers which have been homogenized at room temperature.

### DESCRIPTION OF ILLUSTRATIVE EMBODIMENTS

Acellular tissue matrix tissue is the result of a multistep process in which the tissue is collected from a donor and processed so as to isolate the natural tissue matrix. In its preferred form, the process includes the steps of processing biological tissues including treatment with a stabilizing solution to reduce procurement damage, treatment with a processing solution to remove cells and other antigenic tissue components, treatment with a cryoprotectant solution, freezing and storage under specific conditions to avoid functionally significant damaging ice crystal formation, drying under conditions to prevent damaging ice recrystallization, storage in the dry state at above freezing temperatures, rehydration under specific conditions and with a rehydration solution to minimize surface tension damage and further augment the selective preservation of the matrix, and reconstitution with viable cells that will not be rejected by the host.

The above summarized process for producing acellular dermal or other tissue matrix is more fully disclosed in U.S. Patent Number 5,336,616.

One of skill in the art will appreciate however that other acellular tissue matrix tissues may be used in the present invention. For example, acellular tissue matrix tissue derived from xenogenic source may be used in addition to the human derived tissue disclosed above, and other tissues such as blood vessels, heart valves, fascia and nerve connective tissue may be used to create a particulate acellular matrix according to the present invention. Thus in one preferred embodiment of the present invention, the method disclosed herein utilizes acellular tissue matrix tissue, herein also referred to as AlloDerm^{®}, which is commercially available from LifeCell Corporation, The Woodlands Texas.

The process of the present invention utilizes a chemical free and minimally disruptive technique which minimizes the damage to the collagen fibers including sheared fiber ends that result from the conventional wet or dry processes previously disclosed. The resulting particulate acellular tissue matrix can be suspended in a suitable carrying agent and thereby is made suitable for delivery through hypodermic needle injection or other modes of application including, spraying, layering, packing, in-casing or combinations of these methods. One of skill in the art should also appreciate that the particulate acellular matrix may be reconstituted into a sheet, or into a gelatinous form or other forms for use.

Generally the method of the present invention includes the steps of: cryofracturing the dry acellular tissue matrix strips at cryogenic temperatures; and separating the resulting particles by size at cryogenic temperatures. In a preferred embodiment, the method of the present invention includes: cryofracturing the dry acellular tissue matrix strips at cryogenic temperatures; separating the resulting particles by size at cryogenic temperatures; and freeze drying the fraction of particles desired size to remove any moisture that may have been absorbed to give a dry particulate acellular tissue matrix. In a more preferred embodiment the method of the present invention includes: cutting sheets of dry acellular tissue matrix into strips; cryofracturing the dry acellular tissue matrix strips at cryogenic temperatures; separating the resulting particles by size at cryogenic temperatures; freeze drying the fraction of particles desired size to remove any moisture that may have been absorbed to give a dry particulate acellular tissue matrix; and rehydrating the dry particulate acellular tissue matrix.

Preferably the dry acellular tissue matrix is cryogenically cooled to a temperature that permits the cryogenic fracturing, shattering or milling of the material. In one embodiment of the present invention a sterilized homogenizer cooled to liquid nitrogen temperatures is utilized. The resulting cryogenically fractured material is then passed through a series of particle size exclusion screens so as to isolate the desired range of particulate acellular tissue matrix material. Once isolated, the particulate acellular tissue matrix material may be freeze-dried so as to remove any moisture that may have been absorbed to the matrix during the above described process.

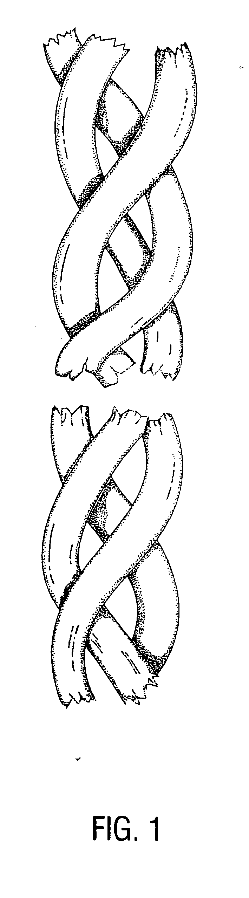

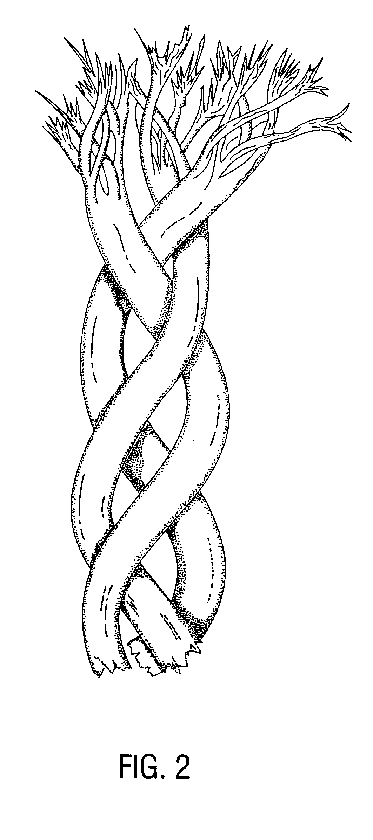

Generally the particulate acellular tissue matrix is produced in such a way as to minimize the amount of mechanical damage incurred when reducing an intact tissue to a particulate form. As illustrated in Fig. 1. the cryofracturing of the collagen fibers in accordance with the present invention results in a "clean" break of the collagen fibers. This is in contrast with the frayed ends and substantially damaged collagen fibers that result from the room temperature shredding of collagen tissue as is the practice in the prior art. The damage cause by the mechanical shredding of the collagen fiber bundle, and in particular the end of the collagen fibers is illustrated in Fig. 2. The above illustrations are based on electron microscopic observation of the processed materials and are representative of the ends of the collagen fiber bundles present in the collagen based tissues.

In developing the process of the present invention, several factors were found to be important to the new and unexpected properties of the resulting particulate acellular tissue matrix. One such factor is the temperature at which the homogenization or cryofracturing of the dry acellular matrix, such as AlloDerm^{®}, takes place. As the term is used herein, homogenization and cryofractuing are utilized interchangably and are intended to mean the process of creating particulate material from the sheet like starting material. It has been found that the temperature at which the cryofractuing takes place should be sufficiently enough below room temperature so that the collagen fibers are cryogenically fractured and not shredded or torn. Generally the temperature should be below 0° C and preferably the temperature should be below -50 °C and more preferably should be below -100° C. Commercially available refrigerants can be used to achieve such temperatures, which may include halocarbon refrigerants, liquid carbon dioxide, liquid nitrogen, liquid argon, liquid helium and other similar such well known non-chemically reactive and thus inert and non-toxic refrigerants.

Another factor found to be important in achieving the present invention is the particle size of the particulate acellular tissue matrix material. In order to select the desired range of particles, a cryogenically cooled homogenizing tower is utilized. The role of the homogenization tower is to separate particles which are too small or too large in size from those within the desired particle size range. In one embodiment, liquid nitrogen is utilized in combination with a first metal screen to reject those particles that are too large. A second metal screen is used in conjunction with liquid nitrogen to capture those particles of the proper minimum size and to allow those particles that are too small to be removed. In one preferred embodiment the first screen is (0.03 inch) 0.0762 cm metal screen and the second screen is (0.0015 inch) 0.00381 cm metal screen. In another embodiment the screens are selected so as to isolate particles having a size of 1 µm (microns) to 900 µm (microns) and preferably screens are selected to isolate particles having a size from 30 µm (microns) to 800 µm (microns).

The resulting particulate acellular tissue matrix may be rehydrated by suspension of the particles in any suitable aqueous solution, preferably normal saline and local anesthetic. If desired the rehydrated particulate acellular tissue matrix may be isolated by filtration or pelletizing the particle via centrifugation and decantation of the supernatant. The particulate acellular tissue matrix may then be resuspended to an appropriate concentration in a suitable physiologically compatible carrier, such as normal saline, normal saline and local anesthetic or if desired a pharmaceutical carrier. Either the physiological carrier or the rehydrating saline solution may contain antibiotics or other drugs, cells or cell extracts, anti-inflammatory agents, proteoglycans, analgesics, hemostatic agents, growth factors such as epidermal growth factor, fibroblast growth factor, nerve growth factor, keratinocyte growth factor, platelet derived growth factor, vasoactive intestinal peptide, stem cell factor, bone morphogenic proteins, chondrocyte growth factor and other similar such components as well as other components that are desirable at the injection site.

Once rehydrated, the particulate acellular tissue matrix may also be combined with stem or progenitor cells of non human embryonic origin prior to or during transplantation into the host. These stem cells may be native to the site of transplantation, but due to their nature need not be. One of ordinary skill in the art should understand and appreciate that upon division, stem cells replicate and also give rise to cells that differentiate further into one or more specialized cells. Such stem cells may include mesenchymal stem cells, epidermal stem cells, cartilage stem cells, hematopoietic stem cells, and other similar cells.

Thus in one illustrative embodiment of the present invention the processing of acellular tissue matrix to create injectable size particles includes: cutting sheets of dry acellular tissue matrix into strips using a modified cutting or meshing devise; homogenization of the dry acellular tissue matrix strips at cryogenic temperatures; separation of the resulting particles by size at cryogenic temperatures; and freeze drying the fraction of particles desired to remove any moisture that may have been absorbed during homogenizing.

Another illustrative embodiment of the present invention includes the processing of acellular tissue derived from a human donor, e.g. AlloDerm^{®}, to create injectable size particles includes: cutting sheets of dry acellular tissue matrix into strips using a modified cutting or meshing devise; equilibration of homogenizing equipment to liquid nitrogen temperatures; addition of dry acellular tissue matrix strips to a liquid nitrogen cooled homogenizer; cryofracturing of the dry acellular tissue matrix strips at liquid nitrogen temperatures; separation of the resulting particles by size at liquid nitrogen temperatures; and freeze drying the fraction of particles desired to remove any moisture that may have been absorbed during homogenizing.

Potential applications of the particulate acellular tissue matrix materials disclosed herein may include: dermatological applications such as acne scar revision, replacement of dermis lost to disease or accident; urological applications such as the relief of incontinence, and vesicoureteral reflux; otolaryngological applications including vocal cord position adjustment; reconstructive surgical applications to replace tissue lost to cancer surgery or other surgical procedures in which there is the removal of tissue; cosmetic surgery procedures such as tissue replacement, reconstruction or augmentation procedures; correctional procedures for gastrointestinal reflux; and other applications which should be appreciated by one of skill in the art. As an illustrative example, see U.S. Patent No. 5,712,252

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result.

### Preparation Of Acellular Tissue Matrix

The following procedure was carried out in accordance with the teachings of process for producing acellular dermal or other tissue matrix is fully disclosed in U.S. Patent Number 5,336,616, as well as being the subject of co-pending U.S. Patent Applications including: 09/029,179, filed August 22, 1998 . This material is commercially available from LifeCell Corporation, The Woodlands Texas.

Donor skin is harvested under aseptic conditions with a dermatome, and maintained at 4°C in RPMI 1640 tissue culture media containing penicillin and streptomycin solution for no more than 7 days prior to further processing. Transportation to Life Cell's tissue processing center is via overnight delivery, on wet ice, in the same media. On arrival at the processing center, the temperature of the tissue container is verified to be at least 4°C, or the skin discarded. Following verification of container temperature, donor identification and test screening data, the skin is transferred to a laminar-flow hood for further processing.

The donor skin is removed from the transportation container and placed with its reticular side down on a piece of sizing support being a low density polyethylene. An appropriately sized piece of gauze is added to the epidermal side of the skin which is then cut into a rectangular piece as large as possible, not to exceed a 25.8x25.8 cm square (4 x 4 inch square) and no smaller than 2.1x7.6 cm (2 x 3 inches.) The skin is then placed reticular side down, in a petri dish, to which 50 ml of De-epidermizing Solution consisting of 1M NaCl is added. The petri dish is then transferred to an incubator and incubated at 37°±2°C for 18 to 32 hours for human skin and 35 to 55 hours for porcine skin.

After incubation, the petri dish containing the skin is transferred to a laminar flow hood for deepidermization. The gauze is first removed and discarded. The epidermis is then gently grasped with forceps and pulled away from dermis as a sheet. The excess De-epidermizing Solution is then aspirated. A slit approximately one centimeter long is then made in the lower left corner of the dermis to identify the upper and lower surfaces.

The dermis is next rinsed in the same petri dish by the addition of 50 ml Tissue Wash Solution, consisting of sterile Hanks' balanced salt solution. The petri dish is then placed on a rotator at 40±5 RPM for 5 minutes at room temperature (20°-26°C). The petri dish is then returned to the laminar flow hood and the lid from the petri dish is removed in order to aspirate the Tissue Wash Solution. This procedure is repeated a further two times.

The dermis is then treated with 50 ml, of De-cellularizing solution and the petri dish is placed on a rotator at 40±5 RPM for 1 hour at room temperature (20°-26°C). The decellularizing solution for human skin consists of 0.5% sodium dodecyl sulfate in Hanks' balanced salt solution and for porcine skin contains 1 mM disodium ethylenediamine tetraacetic acid (EDTA). The De-cellularizing solution is removed by aspiration. The dermis is then washed with 50 ml of Tissue Wash Solution. The petri dish is then placed on a rotator at 40±5 RPM for 5 minutes at room temperature (20°-26°C). The Tissue Wash Solution is removed by aspiration. The washing procedure is repeated (2) times. After the dermis has been washed a total of 3 times 50 ml of Pre-freezing Solution is added to the petri dish. The dish is then placed on a rotator at 40±5 RPM for 30 minutes at room temperature (20°-26°C). The prefreezing solution for human skin consists of 7% dextran (70,000 MWT), 6% sucrose, 6% raffinose and 1 mM disodium ethylenediamine tetraacetic acid in Hanks' balanced salt solution. The prefreezing solution for porcine skin consists of 7.5% dextran (70,000 MWT), 6% sucrose, 7.5% polyvinylpyrrolidone (MWT 40,000), 1.25% raffinose and 1 mM disodium ethylenediamine tetraacetic acid made up in Hanks' balanced salt solution.

A new piece of gauze is then placed on the papillary side of the dermis and the dermis is turned over so that the reticular side faces up. The backing from the reticular side of the piece of dermis is discarded into a biohazard waste container. An approximately 0.5 to 1.0 cm wide strip of backing and dermis is then cut from the original sample. This strip is then cut into two satellite pieces, each approximately 1.0 cm long. All necessary quality assurance is ultimately performed on these satellite samples, including microbiology and structural analysis.

The tissues are then transferred into individual TYVEK bags. The tissues are positioned in the bag backing side up with the white vent side drown. The TYVEK bag is then heat sealed.

The sealed Freeze-dry Bag is transferred to a freeze-dryer which has a minimum shelf temperature of -70°C and a minimum condenser temperature of -85°C. The tissue is then frozen on the freeze-dryer shelf by ramping the shelf temperature at a rate of -2.5°C/minute to -35°C, and held for at least 10 minutes.

The drying cycle is such that the final residual moisture content of the sample is less than 6% and optimally 2%. In this example, the frozen dermis is dried by the following program:
1. The shelf temperature is ramped at a rate of -2.5°C/minute to -35°C, and held for 10 minutes, with vacuum set to (2000mT) 266 Pa.
2. The shelf temperature is then ramped at a rate of 1.5°C/minute to -23°C, and held for 36 hours with vacuum set to (2000mT) 266 Pa.
3. The temperature is then ramped at rate of 1.5°C/minute to a shelf temperature of - 15°C, and held for 180 minutes with vacuum set to (2000mT) 266 Pa.
4. The temperature is then ramped at a rate of 1.5°C/minute to a shelf temperature of -5°C and held for 180 minutes with vacuum set to (2000mT) 266 Pa.
5. The temperature is finally ramped at a rate of 1.5°C/minute to a shelf temperature of 20°C and held for 180 minutes with the vacuum set to (0 mT) 0 Pa.

Following drying, the Freeze-dry Bag containing the dried dermis is unloaded under an atmosphere of dry nitrogen gas, placed in a second predried impervious pouch and heat sealed under the same inert environment.

During the processing procedure and prior to sealing for freeze drying, a satellite sample is cut from the main sample and further processed under identical conditions to the main sample. Prior to use of the main sample in transplantation, all necessary quality assurance is performed on the satellite sample, including microbiology and structural analysis.

Following drying, the sample is stored at above freezing temperatures, optimally 4°C in a light protected environment.

### Preparation Of Particulate Acellular Tissue Matrix

The following procedure utilizes AlloDerm^{®}, an acellular tissue matrix packaged without a backing material the preparation of which is described above. AlloDerm^{®} is commercially available from LifeCell Corporation, The Woodlands Texas. After removal from the packaging, the dry, acellular tissue matrix is cut into strips using a Zimmer mesher fitted with a non-interrupting "continuous" cutting wheel. The resulting long strips of acellular tissue matrix are cut into lengths of 1 to 2 centimeters in length.

A homogenizer and sterilized homogenizer probe, such as a LabTeck Macro homogenizer available from OMNI International, Warrenton VA, is assembled and cooled to cryogenic temperatures using sterile liquid nitrogen which is poured into the homogenizer tower. Once the homogenizer has reached cryogenic temperatures, acellular tissue matrix previously prepared into strips as noted above are added to the homogenizing tower containing sterile liquid nitrogen. The homogenizer is then activated so as to cryogenically fracture the strips of acellular tissue matrix. The time and duration of the cryogenic fractionation step will depend upon the homogenizer utilized, the size of the homogenizing chamber, the speed and time at which the homogenizer is operated and should be able to be determined by one of skill in the art by simple variation of the parameters to achieve the desired results.

The cryofractured particulate acellular tissue matrix material is sorted by particle size by washing the product of the homogenizer with liquid nitrogen through a series of metal screens, that have also been cooled to liquid nitrogen temperatures. We have found it especially useful to utilize a combination of screens within the homogenizing tower of the type described above in which the particles are washed and sorted first to exclude oversized particles and then to exclude undersized particles.

Once isolated, the particulate acellular tissue matrix is removed and placed in a vial for freeze drying once the sterile liquid nitrogen has evaporated. This last step is to ensure that any residual moisture that may have been absorbed during the above procedure is removed.

The final product is a white powder having a particle size of 1 µm (micron) to 900 µm (microns) and preferably a particle size of 30 µm (microns) to 750 µm (microns). Preferably the particles are distributed about a mean of 150-300 µm (microns). The material is readily rehydrated by suspension in normal saline or other similar suitable rehydrating agent. The rehydrated acellular tissue matrix may be resuspended in normal saline or any other suitable pharmaceutically compatible carrier.

Injection Of Particulate Acellular Tissue Matrix: A sample of the dry cryofractured particulate acellular tissue matrix made in accordance with the procedure disclosed hereinabove was rehydrated and resuspended in phosphate buffer saline at a concentration of 50 mg of particulate material per milliliter of phosphate buffered saline. The suspension was drawn into 1 cc tuberculin syringes. The samples were sent by overnight delivery at 4° C to an independent laboratory for injection into test animals. The samples were injected either into the dorsum of the back in the subcutaneous plane or subauricularly (on the back of the ears) of rats. The animals were monitored for 3 days, at which time samples of the skin surrounding and including the area of injection were excised for evaluation. Histological evaluation revealed particles of human dermis just above the subcutaneous muscle layer in the rat. Microscopic examination of the excised tissue revealed that the particulate acellular tissue matrix had been repopulated with rat cells with no evidence of severe acute inflammatory response by the host animal.

Comparison Of Wet Processed And Cryofractured Acellular Tissue Matrix: Samples of both the wet processed (room temperature) particulate acellular tissue matrix and the cryofractured (liquid nitrogen temperature) particulate acellular tissue matrix were prepared in the following manner: Wet processed particulate acellular tissue matrix was made by first rehydrating a sample of AlloDerm^{®} and cutting the rehydrated acellular tissue matrix into strips and homogenizing those strips in phosphate buffered saline at room temperature. The cryofractured particulate acellular tissue matrix was prepared in accordance with the process of the present invention as described hereinabove. Prior to use, the cryofractured particulate acellular tissue matrix was rehydrated in phosphate buffered saline and pelleted using centrifugation.

Samples of each of the resulting particulate acellular tissue matrix materials were suspended in phosphate buffer saline at a concentration of about 50 mg of particulate material per milliliter of phosphate buffered saline. The suspension was drawn into 1 cc tuberculin syringes. The samples were sent by overnight delivery at 4° C to an independent laboratory for injection into test animals. Samples were injected either into the dorsum of the back in the subcutaneous plane or subauricularly into rats. The animals were monitored for 3 weeks after which samples of the tissue surrounding and including the point of injection were excised for evaluation. Macroscopic and microscopic inspection of the samples revealed the following:

| | Wet Processed | Cryofractured |
|---|---|---|
| Macroscopic | 1/15* | 12/15* |
| Microscopic | 6/30 * | 24/30* |

| | | |
|---|---|---|
| * # of samples exhibiting persistence of particulate AlloDerm^{®} / Total # of samples | | |

Upon review of the above, one of skill in the art should understand and appreciate that the samples that were processed in accordance with the present invention had a rate of persistence approximately four times that of the wet processed material. From this, such a person should conclude that the wet processed particulate acellular tissue matrix causes fundamental changes to the physiological properties of the acellular tissue matrix resulting in a more rapid degradation or resorption of the sample by the host animal.

Long Term Animal Study: Cryofractured particulate acelluar porcine tissue matrix prepared in accordance with the process of the present invention was injected subcutaneously and subdermally in a pig model organism. A concentration of 150 mg of particulate matrix per milliliter of saline was used in this study. Biopsies from the injection sites were obtained at one, three and six months post-injection. These biopsies revealed evidence of persistence of the particulate matrix at 6 months with no evidence of acute inflammation. Further, the particulate matrix had become repopulated with porcine fibroblasts and exhibited evidence of revascularization of the particulate matrix.

In view of the above disclosure, one of ordinary skill in the art should appreciate that one illustrative embodiment of the present invention includes a method of processing an acellular tissue matrix to give a particulate acellular tissue matrix, the method including: cryofracturing the dry acellular tissue matrix strips at cryogenic temperatures; and separating the resulting particles by size at cryogenic temperatures so as to produce the particulate acellular tissue matrix.

Another illustrative embodiment of the present invention includes a method of processing an acellular tissue matrix to give a particulate acellular tissue matrix. The illustrative the method includes: cryofracturing the dry acellular tissue matrix strips at cryogenic temperatures; separating the resulting particles by size at cryogenic temperatures; and freeze drying the fraction of particles of desired size to remove any moisture that may have been absorbed so as to produce the particulate acellular tissue matrix.

Yet another illustrative embodiment of the present invention included a method of processing an acellular tissue matrix to give a particulate acellular tissue matrix, the method comprising: cutting sheets of dry acellular tissue matrix into strips; cryofracturing the dry acellular tissue matrix strips at cryogenic temperatures; separating the resulting particles by size at cryogenic temperatures; freeze drying the fraction of particles desired size to remove any moisture that may have been absorbed to give a dry particulate acellular tissue matrix; and rehydrating the dry particulate acellular tissue matrix.

In addition to the above methods, the present invention is also directed to the product of the processes described herein. In one illustrative embodiment the product includes the product of the process disclosed herein along with growth and stimulating agents selected from epidermal growth factor, fibroblast growth factor, nerve growth factor, keratinocyte growth factor, platelet derived growth factor, vasoactive intestinal peptide, stem cell factor, bone morphogenic proteins, chondrocyte growth factor and combinations thereof. Another embodiment of the present invention includes the product of the present invention combined with stem cells of non human embryonic origin selected from mesenchymal stem cells, epidermal stem cells, cartilage stem cells, hematopoietic stem cells and combinations thereof. The product of the present invention may also further include analgesic drugs or hemostatic drugs or antibiotic drugs or combinations of these.

While the compositions and methods of this invention have been described in terms of preferred embodiments, it should be apparent to those of skill in the art that variations may be applied to the process described herein.

## Claims

1. The use of a product consisting in a particulate tissue matrix obtained by cryofracturing an acellular tissue matrix wherein the acellular tissue matrix is made from a collagen-containing tissue and is acellular and dry; so as to give a particulate tissue matrix wherein ends of collagen fibers created by the fracturing are not frayed ends; for the preparation of a medicament for the treatment of a defect selected from the group consisting of an acne scar, urinary incontinence, vesicoureteral reflux, and gastrointestinal reflux, said medicament being in a pharmaceutical form such as can be (a) injected, (b) sprayed, (c) layered, (d) packed, (e) in-cased or (f) combinations of (a) - (e).

2. The use according to claim 1, **characterized in that** the lost tissue is dermis.

3. The use according to claim 1, **characterized in that** the lost tissue is tissue lost in surgery in which tissue is removed.

4. The use according to any of claims 1 to 3, **characterized in that** the acellular tissue matrix is made from a human tissue.

5. The use according to claim 1, wherein the product further comprises growth and stimulating agents selected from the group consisting of: (a) epidermal growth factor, (b) fibroblast growth factor, (c) nerve growth factor, (d) keratinocyte growth factor, (e) platelet derived growth factor, (f) vasoactive intestinal peptide, (g) stem cell factor, (h) bone morphogenic proteins, (i) chondrocyte growth factor, and (j) combinations of (a) - (i).

6. The use according to claim 1, wherein the product further comprises stem cells of non human embryonic origin selected from the group consisting of (a) mesenchymal stem cells, (b) epidermal stem cells, (c) cartilage stem cells, (d) hematopoietic stem cells, and (e) combinations of (a) -(d).

7. The use according to claim 1, wherein the product further comprises analgesic drugs.

8. The use according to claim 1, wherein the product further comprises hemostatic drugs.

9. The use according to claim 1, wherein the product further comprises antibiotic drugs.

10. The use according to claim 1, wherein the dry particulate acellular tissue matrix has a particle size of 1 µm to 900 µm.

11. The use according to claim 1, wherein the dry particulate acellular tissue matrix has a particle size of 30 µm to 800 µm.

12. The use according to claim 1, wherein the acellular tissue matrix is made from dermis.

13. The use according to claim 1, wherein the acellular tissue matrix is made from a tissue selected from the group consisting of blood vessel tissue, heart valve tissue, fascia, and nerve connective tissue.

14. The use according to claim 1, **characterized in that** said particulate tissue matrix is obtained by separating by size at cryogenic temperatures the particles resulting from said cryofracturing step so as to produce the particulate tissue matrix.

15. The use according to claim 14, **characterized by** further comprising, after the separating step:
- a step of freeze drying the fraction of particles of desired size to remove any moisture that may have been absorbed so as to produce the particulate acellular tissue matrix.

16. The use according to claim 15, **characterized by** further comprising prior to said cryofracturing step, a step of cutting sheets of dry acellular tissue matrix into strips; and after said freeze drying step, a step of rehydrating the dry particulate acellular tissue matrix.

## Patentansprüche

1. Verwendung eines Produkts, umfassend eine partikelförmige Gewebematrix, die durch Gefrierbruch einer azellulären Gewebematrix erhalten wird, wobei die azelluläre Gewebematrix aus einem kollagenhaltigen Gewebe hergestellt sowie azellulär und trocken ist, um so eine partikelförmige Gewebematrix zu erhalten, in der durch Bruch erzeugte Enden der Kollagenfasern nicht ausgefranst sind, für die Herstellung eines Medikaments zur Behandlung eines Defekts, der aus der Gruppe bestehend aus Aknenarben, Harninkontinenz, vesikoureteraler Reflux und gastrointestinaler Reflux ausgewählt ist, wobei das Medikament in einer pharmazeutischen Form vorliegt, so dass es (a) injiziert, (b) gesprüht, (c) geschichtet, (d) tamponiert, (e) eingehüllt oder (f) in Kombination von (a)-(e) verwendet werden kann.

2. Verwendung nach Anspruch 1, **dadurch gekennzeichnet, dass** das verlorengegangene Gewebe Haut ist.

3. Verwendung nach Anspruch 1, **dadurch gekennzeichnet, dass** das verlorengegangene Gewebe aus der Chirurgie, bei der Gewebe entfernt wird, herrührt.

4. Verwendung nach einem der Ansprüche 1 bis 3, **dadurch gekennzeichnet, dass** die azelluläre Gewebematrix aus menschlichem Gewebe hergestellt ist.

5. Verwendung nach Anspruch 1, wobei das Produkt ferner Wachstums- und Stimulanzmittel aufweist, die aus der Gruppe bestehend aus: (a) epidermischer Wachstumsfaktor, (b) fibroblastischer Wachstumsfaktor, (c) Nervenwachstumsfaktor, (d) Keratinozytwachstumsfaktor, (e) von einem Plättchen abgeleiteter Wachstumsfaktor, (f) vasoaktives intestinales Peptid, (g) Stammzellenfaktor, (h) knochenbildungsanregende Proteine, (i) Chondrozytwachstumsfaktor und (j) Kombinationen aus (a)-(i) ausgewählt sind.

6. Verwendung nach Anspruch 1, wobei das Produkt ferner Stammzellen aufweist, die nicht menschlichen embryonalen Ursprungs sind und aus der Gruppe bestehend aus (a) mesenchymalen Stammzellen, (b) epidermischen Stammzellen, (c) Knorpelstammzellen, (d) hämatopoietischen Stammzellen und (e) Kombinationen aus (a)-(d) ausgewählt sind.

7. Verwendung nach Anspruch 1, wobei das Produkt ferner analgetische Arzneimittel aufweist.

8. Verwendung nach Anspruch 1, wobei das Produkt ferner hämostatische Arzneimittel aufweist.

9. Verwendung nach Anspruch 1, wobei das Produkt ferner antibiotische Arzneimittel aufweist.

10. Verwendung nach Anspruch 1, wobei die trockene, partikelförmige, azelluläre Gewebematrix eine Partikelgröße von 1 µm bis 900 µm besitzt.

11. Verwendung nach Anspruch 1, wobei die trockene, partikelförmige, azelluläre Gewebematrix eine Partikelgröße von 30 µm bis 800 µm besitzt.

12. Verwendung nach Anspruch 1, wobei die azelluläre Gewebematrix aus Haut hergestellt ist.

13. Verwendung nach Anspruch 1, wobei die azelluläre Gewebematrix aus einem Gewebe hergestellt ist, das aus der Gruppe bestehend aus Blutgefäßgewebe, Herzklappengewebe, Faszie und Nervenbindegewebe ausgewählt ist.

14. Verwendung nach Anspruch 1, **dadurch gekennzeichnet, dass** die partikelförmige Gewebematrix erhalten wird, indem bei kryogenen Temperaturen diejenigen Partikel, die aus dem Gefrierbruch resultieren, nach Größe getrennt werden, um so die partikelförmige Gewebematrix zu erzeugen.

15. Verwendung nach Anspruch 14, **gekennzeichnet durch** einen weiteren Schritt nach dem Trennschritt:
Gefriertrocknen des Anteils der Partikel mit erwünschter Größe, um Feuchtigkeit zu entfernen, die möglicherweise absorbiert worden ist, um so die partikelförmige, azelluläre Gewebematrix zu erzeugen.

16. Verwendung nach Anspruch 15, **gekennzeichnet durch** die weiteren Schritte:
vor dem Gefrierbruch, Schneiden von Lagen der trockenen azellulären Gewebematrix in Streifen, und
nach der Gefriertrocknung, Rehydrieren der trockenen, partikelförmigen, azellulären Gewebematrix.

## Revendications

1. Utilisation d'un produit constitué d'une matrice tissulaire particulaire obtenue par cryofracture d'une matrice tissulaire acellulaire où la matrice tissulaire acellulaire est faite d'un tissu contenant du collagène et est acellulaire et sèche ; de manière à donner une matrice tissulaire particulaire dans laquelle les extrémités des fibres de collagène créées par la fracture ne sont pas des extrémités effilochées ; pour la préparation d'un médicament destiné au traitement d'une anomalie choisie dans le groupe constitué d'une cicatrice d'acné, d'une incontinence urinaire, d'un reflux vésico-urétéral et d'un reflux gastro-intestinal, ledit médicament étant sous une forme pharmaceutique de telle façon qu'il peut être (a) injecté, (b) pulvérisé, (c) déposé en couche, (d) tassé, (e) enveloppé ou (f) des combinaisons de (a) à (e).

2. Utilisation selon la revendication 1, **caractérisée en ce que** le tissu perdu est du derme.

3. Utilisation selon la revendication 1, **caractérisée en ce que** le tissu perdu est du tissu perdu en chirurgie où du tissu est prélevé.

4. Utilisation selon l'une quelconque des revendications 1 à 3, **caractérisée en ce que** la matrice tissulaire acellulaire est faite d'un tissu humain.

5. Utilisation selon la revendication 1, où le produit comprend en outre des agents de croissance et stimulateurs choisis dans le groupe constitué par : (a) le facteur de croissance des cellules épidermiques, (b) le facteur de croissance des fibroblastes, (c) le facteur de croissance des cellules nerveuses, (d) le facteur de croissance des kératinocytes, (e) le facteur de croissance d'origine plaquettaire, (f) le peptide intestinal vasoactif, (g) le facteur des cellules souches, (h) les protéines morphogéniques osseuses, (i) le facteur de croissance des chondrocytes et (j) des combinaisons de (a) à (i).

6. Utilisation selon la revendication 1, où le produit comprend en outre des cellules souches d'origine embryonnaire non humaine choisies dans le groupe constitué de (a) cellules souches mésenchymateuses, (b) cellules souches épidermiques, (c) cellules souches cartilagineuses, (d) cellules souches hématopoïétiques et (e) des combinaisons de (a) à (d).

7. Utilisation selon la revendication 1, où le produit comprend en outre des médicaments antalgiques.

8. Utilisation selon la revendication 1, où le produit comprend en outre des médicaments hémostatiques.

9. Utilisation selon la revendication 1, où le produit comprend en outre des médicaments antibiotiques.

10. Utilisation selon la revendication 1, où la matrice tissulaire acellulaire particulaire sèche a une taille de particule de 1 µm à 900 µm.

11. Utilisation selon la revendication 1, où la matrice tissulaire acellulaire particulaire sèche a une taille de particule de 30 µm à 800 µm.

12. Utilisation selon la revendication 1, où la matrice tissulaire acellulaire est faite de derme.

13. Utilisation selon la revendication 1, où la matrice tissulaire acellulaire est faite d'un tissu choisi dans le groupe constitué de tissu de vaisseau sanguin, tissu de valve cardiaque, tissu conjonctif de fascia et de nerf.

14. Utilisation selon la revendication 1, **caractérisée en ce que** ladite matrice tissulaire particulaire est obtenue en séparant par taille à des températures cryogéniques les particules résultant de ladite étape de cryofracture de manière à produire la matrice tissulaire particulaire.

15. Utilisation selon la revendication 14, **caractérisée en ce qu'**elle comprend en outre, après l'étape de séparation :
une étape de cryodessication de la fraction des particules de la taille souhaitée pour éliminer toute humidité qui peut avoir été absorbée de manière à produire la matrice tissulaire acellulaire particulaire.

16. Utilisation selon la revendication 15, **caractérisée en ce qu'**elle comprend en outre avant ladite étape de cryofracture, une étape consistant à couper des feuillets de la matrice tissulaire acellulaire sèche en bandelettes ; et après ladite étape de cryodessication, une étape consistant à réhydrater la matrice tissulaire acellulaire particulaire sèche.
